Europäisches Patentamt

European Patent Office

Office européen des brevets

(11) Veröffentlichungsnummer: **0 216 095**
**A2**

# (12) EUROPÄISCHE PATENTANMELDUNG

(21) Anmeldenummer: 86110876.9

(51) Int. Cl.⁴: **A61B 17/22**

(22) Anmeldetag: 06.08.86

(30) Priorität: 19.08.85 DE 8523751 U

(43) Veröffentlichungstag der Anmeldung:
01.04.87 Patentblatt 87/14

(84) Benannte Vertragsstaaten:
DE FR GB

(71) Anmelder: **Siemens Aktiengesellschaft Berlin und München**
**Wittelsbacherplatz 2**
**D-8000 München 2(DE)**

(72) Erfinder: **Wessels, Gerd, Dr.**
**Tetzelweg 42**
**D-8520 Erlangen(DE)**
Erfinder: **Rohwedder. Arnim, Dipl.-Ing.**
**Osloerstrasse 6**
**D-8520 Erlangen(DE)**

(54) **Vorrichtung für die Beschallung von pathologischen Veränderungen in einem Patienten.**

(57) Die Erfindung betrifft eine Vorrichtung für die Beschallung von pathologischen Veränderungen in einem Patienten. Ein Gehäuse (1) enthält einen Stoßwellengenerator (5), der Stoßwellen durch einen von einer Membran (3, 3', 3") abgeschlossenen, flüssigkeitsgefüllten Raum (4) aussendet. Auf ihm ist ein Ring (11) aus elastischem Material befestigt, der die Membran (3, 3', 3") umschließt und im Ruhezustand über diese hinausragt. Im Ring ist ein Kanalsystem zum Evakuieren der Luft aus dem Raum zwischen Ring (11), Membran (3, 3', 3") und der Hautoberfläche (2) und zum Einführen einer Koppelflüssigkeit vorgesehen.

EP 0 216 095 A2

## Vorrichtung für die Beschallung von pathologischen Veränderungen in einem Patienten

Die Erfindung betrifft eine Vorrichtung für die Beschallung von pathologischen Veränderungen in einem Patienten mit einem Stoßwellengenerator, der Stoßwellen durch einem von einer Membran abgeschlossenen, flüssigkeitsgefüllten Raum aussendet.

Ein Gerät dieser Art ist z. B. in der DE-AS 23 51 247 beschrieben. Es handelt sich dabei um eine Vorrichtung, wie sie für die Zertrümmerung von Harn-, Nieren-und Gallensteinen dient. Im Innenraum einer durch eine elastische Membran abgeschlossenen Fokussierungskammer werden mit Hilfe einer Funkentladungsstrecke Stoßwellen über eine in der Kammer befindliche Koppelflüssigkeit an die Membran übertragen. Die Form der Fokussierungskammer bildet einen Teil eines Rotationsellipsoids, in dessen einem Brennpunkt der Stoßwellenerzeuger liegt. Die von ihm erzeugten Stoßwellen werden durch die Wandungen der Fokussierungskammer in den anderen Brennpunkt des Rotationsellipsoids fokussiert, der außerhalb der Kammer liegt. Für die Durchführung der Zertrümmerung müssen u.a. folgende zwei Bedingungen erfüllt sein:

a) Der außerhalb der Fokussierungskammer liegende zweite Brennpunkt des Rotationsellipsoids muß beim Aufliegen der Kammer auf der Haut des Patienten mit der Lage des Konkrementes zusammenfallen.

b) Zwischen der Membran und der Haut des Patienten sollen sich keine Luftzwischenräume befinden, da diese wegen ihres stark abweichenden Schallwiderstandes eine gute Einkopplung der Wellen verhindern würden (Reflexionsanteil wird höher).

Aufgabe der vorliegenden Erfindung ist es, die beim Anlegen der eingangs genannten Vorrichtung zwischen Membran und Haut des Patienten eingeschlossenen Luftzwischenräume zu entfernen.

Diese Aufgabe ist erfindungsgemäß dadurch gelöst, daß auf dem Gehäuse ein manschettenförmiger Ring aus elastischem Material befestigt ist, der die dehnbare Membran umschließt und im Ruhezustand über diese hinausragt, und daß im Ring ein Kanalsystem zum Evakuieren der Luft aus dem Raum zwischen Ring und Membran und zum Einführen einer Koppelflüssigkeit vorgesehen ist.

Beim Aufsetzen der erfindungsgemäßen Vorrichtung auf den Patientenkörper kommt die Membran im Ruhezustand aufgrund des vorstehenden manschettenförmigen Ringes mit der Haut noch nicht in Berührung. Der Ring, die Membran und die Haut bilden eine abgeschlossene Kammer, die jedoch mit dem im Ring enthaltenen Kanalsystem in Verbindung steht.

Die Luft in der Kammer, gebildet durch Haut, Ring und Membran, wird zunächst teilweise evakuiert. Der dadurch in der Kammer erzeugte Unterdruck hat zur Folge, daß die an der Hautoberfläche (besonders innerhalb der Poren) eingeschlossenen Luftzwischenräume weitgehend reduziert werden. Anschließend kann durch spezielle Kanäle des Systems eine Koppelflüssigkeit in die Kammer eingespritzt werden, die eine Benetzung der Wandungen (Hautoberfläche, Innenwand des Ringes, Außenfläche der elastischen Membran) bewirkt. Während der Benetzung wird die Kammer wei ter evakuiert, wobei sich infolge des Unterdruckes die Membran auswölbt und sich vom Zentrum beginnend nach aussen hin an die Haut anlegt.

Auf diese Weise wird ein Einschließen der in der Kammer noch vorhandenen Luft durch die sich auf die Haut anlegende Membran verhindert.

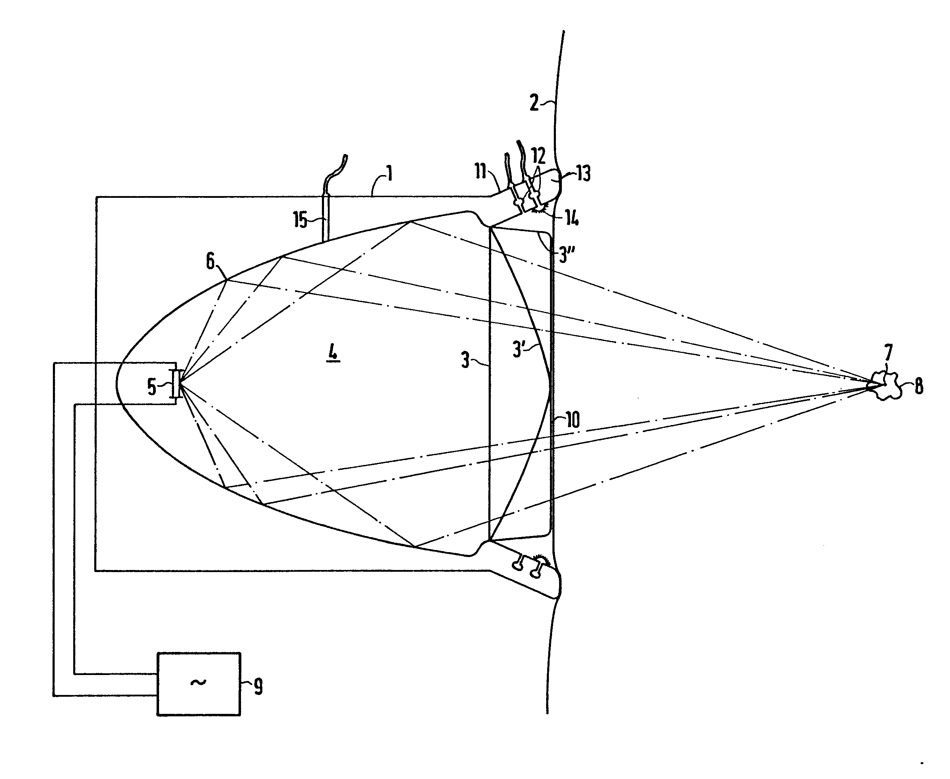

Die Erfindung ist nachfolgend anhand eines in der Zeichnung dargestellten Ausführungsbeispieles näher erläutert.

Die Figur zeigt, wie die Vorrichtung an einem Patienten 2 angesetzt ist. Eine Gehäuse 1 umgibt eine Fokussierungskammer 4, welche mit einer Koppelflüssigkeit, z.B. Wasser, gefüllt und durch eine dehnbare Membran 3 abgeschlossen ist. Im Brennpunkt der Fokussierungskammer ist eine Stoßwellengenerator 5 positioniert, der Stoßwellen auf die ihn umgebende Koppelflüssigkeit überträgt. Der Generator arbeitet nach dem in der DE-AS 23 51 247 beschriebenen Prinzip, also mit Hilfe einer Funkentladung, welche durch eine Versorgungseinheit 9 generiert wird. Die erzeugten Stoßwellen laufen zunächst in der Koppelflüssigkeit vom Generator ausgehend durch die Fokussierungskammer 4, werden teilweise an der Innenwandung 6 reflektiert, gelangen über die elastische Membran 3 bzw. 3' bzw. 3" sowie über die später noch beschriebene, benetzende Koppelflüssigkeit 10 in den Körper des Patienten 2 und werden im Punkt 7 fokussiert. Der Brennpunkt 7 befindet sich innerhalb eines zu zertrümmernden Konkrementes 8.

Der auf dem Gehäuse 1 der Fokussierungskammer 4 befestigte und das Kanalsystem 12 enthaltende, z.B. aus Moosgummi gefertigte Ring 11 besitzt einen dem Patienten 2 zugewandten anschmiegsamen Rand 13, der eventuell auch vor geformt sein kann. Das Kanalsystem 12 besitzt sowohl Kanäle, die die zwischen der Membran 3 und der Haut des Patienten 2 eingeschlossene Luft absaugen als auch Kanäle zum Einspritzen von

beliebiger Koppelflüssigkeit für das Benetzen der Haut und der Membran 3. Letztere Kanäle können zu diesem Zweck z. B. mit feinen Düsen 14 versehen sein. Über einen verschließbaren Kanal 15 kann Koppelflüssigkeit in die Fokussierungskammer 4 nachgelassen werden.

Die beispielsweise aus Latex gefertigte elastische Membran hat ihrem Ruhezustand die mit 3 bezeichnete Lage. Die Anpassung der Vorrichtung an den Patienten geschieht durch folgendes Verfahren:

Bei geschlossenem Kanal 15 wird zunächst der zwischen Membran 3, Ring 11 und Haut 2 des Patienten eingeschlossene Raum evakuiert. Der hierdurch in diesem Raum erzeugte Unterdruck öffnet die Poren der Haut, so daß die dort eingeschlossene Luft entweicht bzw. die Restluft in den Poren ebenfalls Unterdruck aufweist.

In diesem Zustand läßt man eine Koppelflüssigkeit über das Kanalsystem 12 und die Düsen 14 in den evakuierten Raum spritzen, um die Haut des Patienten und die Membranoberfläche mit ihr zu benetzen.

Durch andere Kanäle des Kanalsystems 12 wird dabei weiterhin Luft aus dem Raum evakuiert, um den herrschenden Unterdruck aufrechzuerhalten. Ist eine hinreichend gute Benetzung der Haut erfolgt, werden die Kanäle des Kanalsystems 12 geschlossen und der Kanal 15 zur Fokussierungskammer 4 geöffnet. Die durch ihn eintretende Koppelflüssigkeit baut in der Fokussierungskammer wieder Normaldruck auf und wölbt die Membran 3 in zunehmendem Maße der Hautoberfläche entgegen. Da das Zentrum der Membran sich am weitesten aus der Ruhelage 3 entfernt, wird es zuerst die mit Koppelflüssigkeit benetzte Hautoberfläche berühren. Die Membran hat dann die mit 3' bezeichnete Lage. Bei fortgesetzter Wölbung der Membran legt sie sich vom Zentrum beginnend auf die mit Koppelflüssigkeit benetzte Haut und treibt gleichzeitig vorhandene Luftzwischenräume radial nach außen ab. Der sich dabei im Raum zwischen Haut und Membran aufbauende Druck preßt die benetzende Koppelflüssigkeit in die durch den ersten Schritt evakuierten Porenhohlräume hinein. Ist die Membran im Endzustand 3'', ist ein großer Teil der Hautoberfläche mit der Membran bedeckt, nur noch getrennt durch eine dünne Schicht Koppelflüssigkeit 10, die eine Verminderung der Kopplung, bewirkt durch natürliche Unebenheiten der Haut (Schuppen, Haare), verhindert. Ein verbleibender geringer Unterdruck sorgt zusätzlich für eine feste, stabile Lagerung der Vorrichtung 1 an dem Patienten 2.

Mit Hilfe der beschriebenen Vorrichtung kann also gegenüber dem Stand der Technik eine verbesserte Kopplung der stoßwellenerzeugenden Kammer an den Körper des Patienten erzielt werden, dadurch, daß längs der Einkopplungsfläche keine Luftzwischenräume zwischen Haut und Membran eingeschlossen werden.

Die Realisierung der erfindungsgemäßen Vorrichtung ist nicht beschränkt auf die Verwendung eines Generators, der mit Hilfe einer Funkentladung arbeitet. Alternative Ausführungsformen können auf anderen Prinzipien der Schallerzeugung, z.B. durch Verwendung einer piezokeramischen Scheibe oder eines elektrodynamischen Schaller zeugers. Auch die Benutzung anderer fokussierender Mittel - (Linsen oder Spiegel) ist möglich.

## Ansprüche

Vorrichtung für die Beschallung von pathologischen Veränderungen in einem Patienten mit einem Stoßwellengenerator (5), der Stoßwellen durch eine flüssigkeitsgefüllte Kammer (4) aussendet, welche von einer dehnbaren Membran (3, 3', 3'') abgeschlossen ist, **dadurch gekennzeichnet, daß** auf dem Gehäuse der stoßwellenerzeugenden Kammer (4) ein Ring (11) aus elastischem Material befestigt ist, der die dehnbare Membran umschließt und im Ruhezustand über diese hinausragt, und daß im Ring (11) ein Kanalsystem (12) vorhanden ist, welches zum Evakuieren der Luft aus dem Raum zwischen Ring (11), Membran (3, 3', 3'') und der Hautoberfläche (2) und zum Einführen einer Koppelflüssigkeit dient.

0 216 095